# FASCICULE DE BREVET EUROPEEN

(11) **EP 1 339 347 B1**
(45) Date de publication et mention de la délivrance du brevet: **07.12.2005**
(21) Numéro de dépôt: 01976344.0
(22) Date de dépôt: 01.10.2001
(51) Int. Cl.: A61F 2/00

(54) **ENSEMBLE DE SOUTENEMENT SOUS-URETRAL DANS LE TRAITEMENT DE L'INCONTINENCE URINAIRE D'EFFORT DE LA FEMME**
UNTERSTÜTZUNGSANORDNUNG AN DER UNTERSEITE DES URETERS BEI DER BEHANDLUNG DER INKONTINENZ BEI DER FRAU
SUB-URETHRAL SUPPORTING ASSEMBLY FOR TREATING FEMALE STRESS URINARY INCONTINENCE

(30) Priorité: 05.10.2000 FR 0012750
(43) Date de publication de la demande: 03.09.2003
(73) Titulaire: SOFRADIM PRODUCTION, 01600 Trévoux (FR)
(72) Inventeur: THERIN, Michel, F-69004 LYON (FR)
(74) Mandataire: Guerre, Dominique
(86) Numéro de dépôt international: PCT/FR2001/003027
(87) Numéro de publication internationale: WO 2002/028312

(56) Documents cités:
- EP-A- 1 201 189
- WO-A-00/74633
- WO-A-96/06567
- WO-A-99/62406
- DE-C- 219 999
- US-A- 5 899 909
- US-A- 6 068 591

## Description

La présente invention concerne le traitement chirurgical de l'incontinence urinaire d'effort de la femme.

Le document US 5, 899, 909 décrit un ensemble chirurgical pour soutenir l'urètre chez la femme selon le préambule de la revendication 1, comprenant une bandelette, une gaine entourant la bandelette, et deux aiguilles, chaque extrémité de la bandelette étant fermement attachée à une aiguille.

Le document WO 00 74633, compris dans l'état de la technique selon l'article 54(3) CBE, divulgue un ensemble chirurgical similaire avec des moyens de connexion verrouillables et déverrouillables pour connecter une bande composite à une aiguille.

Plus particulièrement, l'invention concerne un ensemble chirurgical permettant, au terme de l'intervention ou opération, de soutenir l'urètre au moyen d'une bandelette sous-urétrale, dont les deux extrémités sont chacune attachées à une partie appropriée du corps de la patiente, par exemple à la paroi abdominale de celle-ci.

A cette fin, de manière générale, l'ensemble chirurgical considéré par la présente invention, comprend une bandelette de soutènement sous-urétral et une aiguille de traversée percutanée, cette aiguille permettant l'engagement et le tirage de la bandelette de soutènement sous-urétral.

La présente invention a pour objet un ensemble chirurgical tel que précédemment défini, à caractère universel, en ce sens qu'il peut être utilisé, quelle que soit la voie d'abord, et la technique d'intervention retenue par le praticien.

A cette fin, un ensemble chirurgical selon invention présente en combinaison les caractéristiques suivantes :
a) il comprend une bande composite qui rassemble au moins la bandelette de soutènement sous urétrale et une gaine aplatie de protection, ladite bandelette étant disposée, par exemple librement, à l'intérieur de cette gaine ;
b) la gaine aplatie comprend, selon sa longueur, deux parties disposées de part et d'autre d'une zone de séparation, dans laquelle est disposé un moyen de liaison sécable entre les deux dites parties ;
c) la bande composite comprend à chacune de ses deux extrémités une même partie, par exemple une même partie femelle, d'un moyen de connexion, verrouillable et déverrouillable, tandis que le talon proximal de l'aiguille de traversée percutanée comprend une autre partie, par exemple une partie mâle, dudit moyen de connexion.

Pour l'une des voies retenues par le chirurgien, par exemple la voie haute, l'ensemble comprend en outre et éventuellement une pièce plate et souple, atraumatique, de jonction de deux morceaux de la bandelette de soutènement, obtenus par découpe transversale de cette dernière. Et cette pièce de jonction comprend deux moyens opposés de préhension de deux extrémités adjacentes des deux morceaux respectivement de la bandelette de soutènement.

La présente invention est maintenant décrite par référence au dessin annexé, dans lequel :
- La figure 1 représente, vu de face, un ensemble chirurgical selon l'invention,
- La figure 2 représente, vu de côté, l'ensemble chirurgical représenté à la figure 1,
- La figure 3 représente, vue en coupe, un moyen de connexion avec l'aiguille de traversée percutanée, appartenant à l'ensemble chirurgical représenté selon les figures 1 et 2,
- La figure 4 représente, vue de dessus, une pièce de jonction de deux morceaux de la bandelette de soutènement, pouvant appartenir à un ensemble chirurgical selon invention,
- La figure 5 représente, vue de côté, la pièce de jonction représentée à la figure 7, avec ses deux moyens opposés de préhension en position ouverte,
- Les figures 6 à 9 représentent les différentes étapes de mise en place ou mise en oeuvre d'un ensemble chirurgical selon l'invention, par la voie basse,
- Les figures 10 à 13 représentent les différentes étapes de mise en en place ou mise en oeuvre d'un ensemble chirurgical selon l'invention, par une voie dite mixte,
- Les figures 14 à 21 représentent les différentes étapes de mise en en place ou mise en oeuvre d'un ensemble chirurgical selon l'invention, par la voie haute.

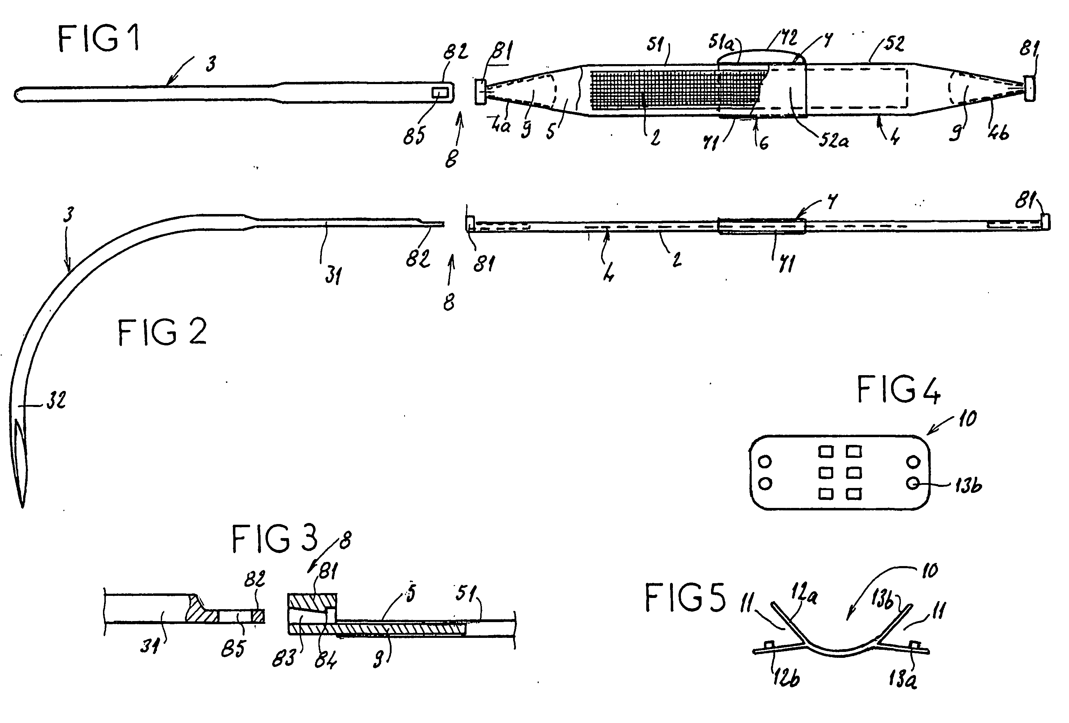

Conformément aux figures 1 à 3, un ensemble chirurgical selon l'invention comprend au minimum :
- une bande composite 4 comportant deux extrémités 4a, 4b effilées, de forme triangulaire, à plat,
- et une aiguille 3 de traversée percutanée comportant une extrémité distale 32 de pénétration et un talon proximal aplati 31

La bande composite 4 rassemble au moins, une bandelette 2 de soutènement sous-urétral, deux embouts 9, et une gaine 5 aplatie de protection, à l'intérieur de laquelle la bandelette 2 est disposée librement, c'est-à-dire sans être attachée à la gaine 5.

La bandelette 2 de soutènement comprend un tricot ajouré, de préférence indémaillable, obtenu avec des monofilaments ou multifilaments en matériau synthétique biocompatible, par exemple en polypropylène ou polyester.

La bandelette 2 est de préférence formée à partir d'un matériau tricoté macro poreux.

Ce dernier est par exemple un tricot ajouré en monofil de polypropylène ayant une grosseur comprise entre 0,12 et 0,16 millimètre et composé de deux nappes formées par deux barres à passettes enfilées chacune, une passette pleine-une passette vide, ces deux barres étant déplacées symétriquement en mailles ouvertes suivant le barème suivant :
- barre I : 01-12-32
- barre II : 32-21-01

La bandelette 2 est découpée en longueur dans le sens de la chaîne du tricot. Dans le cas d'une largeur de 12 mm, elle présente les caractéristiques suivantes :
- une résistance à la rupture dans le sens de la chaîne de 105N ± 20 %,
- un allongement à la rupture dans le sens de la chaîne de 92% ± 20%,
- un allongement sous 20N de 36 %,
- un début de tuilage avec une force de 6N et un allongement de 15 %.

Par "tuilage", on entend l'enroulement spontané de la bandelette 2 sur elle-même, autour de son axe longitudinal, sous contrainte en tension longitudinale.

La bandelette 2 présente des avantages intéressants et en particulier une faible émission de particules lors de son étirement, ainsi qu'un tuilage qui n'apparaît que sous une force importante (6N). Toutes ces caractéristiques précitées n'altèrent en rien la porosité de la bandelette 2.

Cette dernière peut également être réalisée en tout ou en partie en tissu ou matériau biologique, par exemple en collagène.

La gaine de protection 5, aplatie, est obtenu à partir d'un matériau synthétique à faible coefficient de frottement, par exemple en PTFE. Cette gaine 5 comprend, selon sa longueur, deux parties 51 et 52 disposées de part et d'autre d'une zone de séparation 6, dans laquelle est disposé un moyen de liaison 7 sécable entre les deux dites parties.

La bande composite 4 comprend à chacune de ses deux extrémités 4a et 4b une même partie, à savoir une même partie femelle 81, d'un moyen de connexion 8, verrouillable et déverrouillable à volonté, tandis que le talon proximal 31 de l'aiguille 3 de traversée percutanée comprend une autre partie, par exemple une partie mâle 82, du même moyen de connexion 8.

A cette fin, la gaine aplatie 5 comprend ou incorpore, à ses deux extrémités respectivement, deux embouts 9, comprenant ou incorporant, à l'extérieur de la gaine 5, chacun la même partie femelle 81 du moyen de connexion 8.

Ce moyen de connexion 8 comprend donc deux élements, l'un mâle 82 situé à chaque extrémité 4a ou 4b de la bande composite 4, et l'autre femelle 81, situé sur le talon proximal 31 de l'aiguille 3, ces deux éléments mâle et femelle étant encliquetables l'un dans l'autre.

Plus précisemment, comme montré en particulier par la figure 3, le talon proximal 31 de l'aiguille 3 de traversée cutanée est spatulé, et chaque embout 9 de la bande composite 4 comprend, à l'extérieur de la gaine aplatie 5, une fente 83 adaptée pour la pénétration du talon proximal spatulé 31 de l'aiguille 3, avec un moyen 84 de retenue du talon 31, verrouillable ou déverrouillable manuellement. Le talon proximal 31 de l'aiguille 3 comprend une fente traversante 85, pour la pénétration élastique du moyen de retenue 84.

Conformément à la figure 1, et selon un mode particulier de réalisation, le moyen de liaison 7 sécable consiste en un fourreau extérieur 71, adhésif, déchirable, par exemple par cisaillement, au moyen d'un onglet 72. Ce fourreau 71 rassemble deux extrémités intérieures 51a et 52a des deux parties 51 et 52 de la gaine aplatie 5, respectivement adjacentes à la zone de séparation 6.

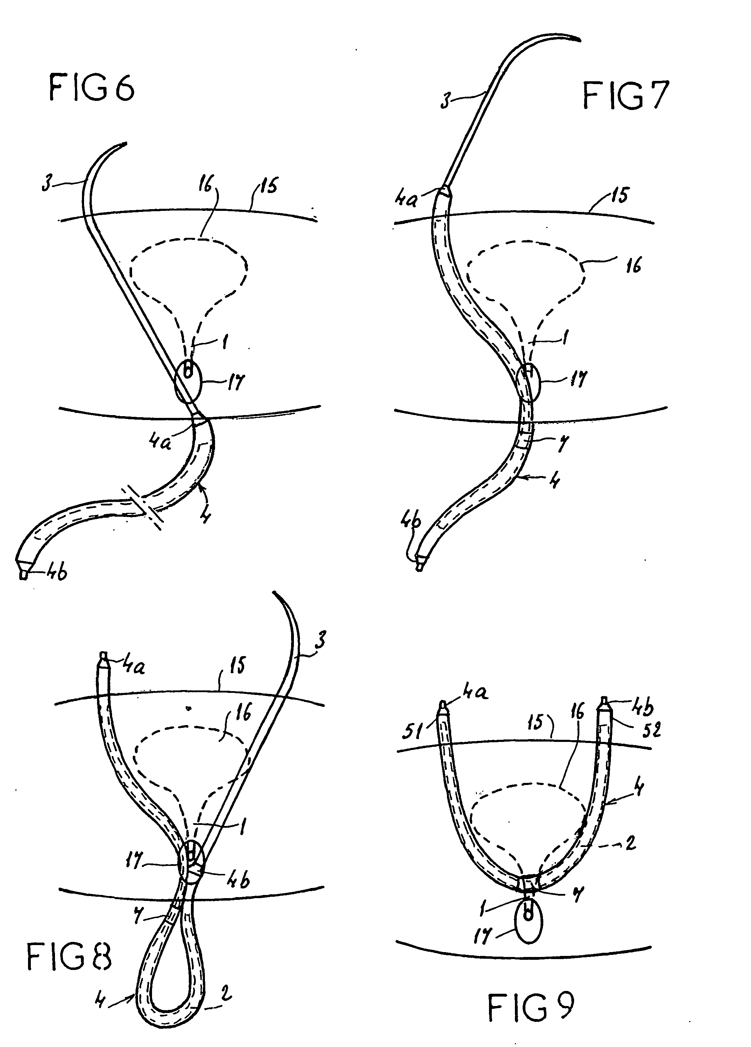

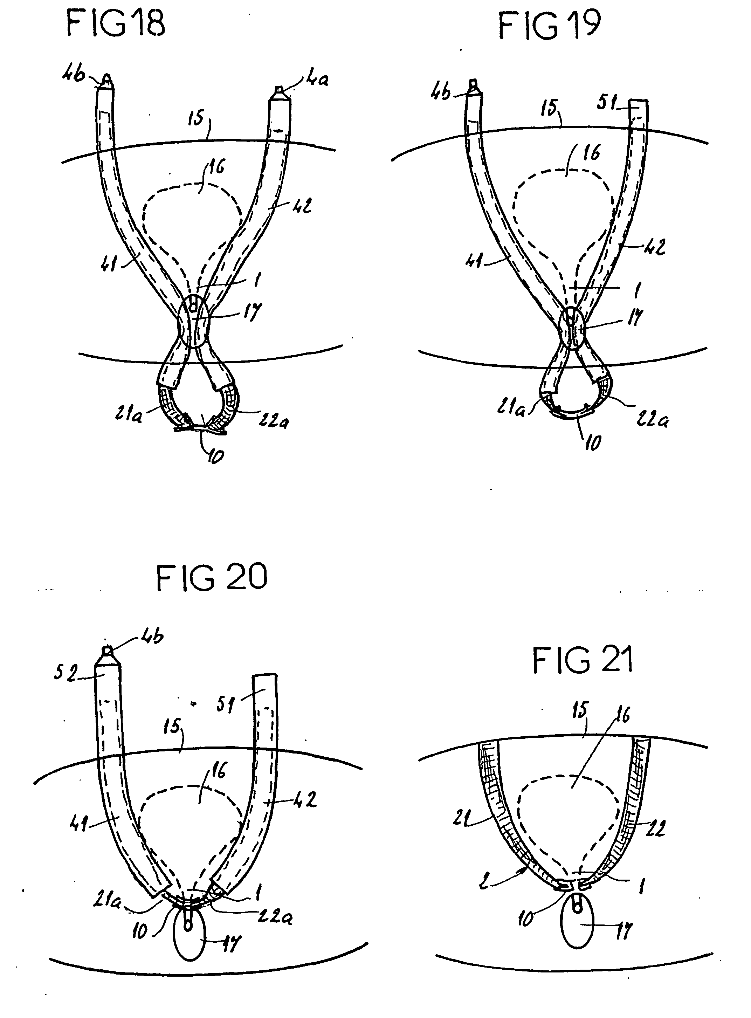

Pour la voie d'intervention dite haute, décrite ci-après, l'ensemble chirurgical comprend en outre une pièce 10 représenté aux figures 4 et 5, adaptée pour joindre deux morceaux 21 et 22 de la bandelette 2 de soutènement (cf figures 18 à 21), obtenus par découpe transversale de cette dernière.

Cette pièce 10 de jonction, atraumatique, plate et souple, comprend deux moyens opposés de préhension, de deux extrémités adjacentes 21a et 22a de deux morceaux 21 et 22 respectivement. A cette fin, chaque moyen 11 de préhension comprend, d'une part un moyen de serrage 12 d'une extrémité 21a ou 22a (cf figures 18 à 21) de la bandelette 2 de soutènement, constitué par deux machoires plates 12a et 12b articulées selon une même ligne de liaison, et d'autre part un moyen 13 d'agrafage de la même extrémité, consistant en un pion 13a sur l'une 12b des machoires, pour traverser la bandelette 2, et une tête 13b disposée sur l'autre machoire 12a, encliquetable sur le pion 13a.

Trois modes différents de mise en place ou mise en oeuvre de l'ensemble chirurgical décrit précédemment sont maintenant exposés, à savoir respectivement un mode dit par voie basse, un mode dit par voie mixte, et un mode dit par voie haute.

Pour chacun de ces modes, on part de l'ensemble chirurgical, selon figures 1 à 3, dans sa position connectée ou verrouillée, dans laquelle l'aiguille 3 est connectée par son talon proximal 31, avec l'un des embouts 9, c'est-à-dire à l'une des extrémités, par exemple 4a, de la bande composite 4, et ce, grâce au moyen de connexion 8 précédemment décrit.

S'agissant du mode dit par voie basse, et en se référant aux figures 6 à 9 :
- en introduisant l'aiguille 3 par le vagin 17, on fait pénétrer l'aiguille 3 de bas en haut, du côté droit du patient, en contournant la vessie 16 ; cf figure 6.
- on engage ainsi la bande composite 4 dans le trajet droit ; cf figure 7.
- on déconnecte l'aiguille 3 de l'extrémité 4a de la bande composite 4, pour la connecter à l'extrémité 4b de la même bande ; puis on fait pénétrer l'aiguille 3 de bas en haut, du côté gauche du patient, toujours en contournant la vessie 16 ; cf figure 8.
- on engage ainsi la bande composite 4 dans le trajet gauche ; lorsque cette dernière forme une boucle à l'extérieur du vagin 17, on déchire transversalement par cisaillement le moyen de liaison 7, pour libérer les deux parties 51 et 52 de la gaine 5 ; en tirant sur les deux embouts 9, on extrait les deux parties 51 et 52 de la gaine 5, et on libère la bandelette de soutènement 2, au-dessous de l'urètre 1.

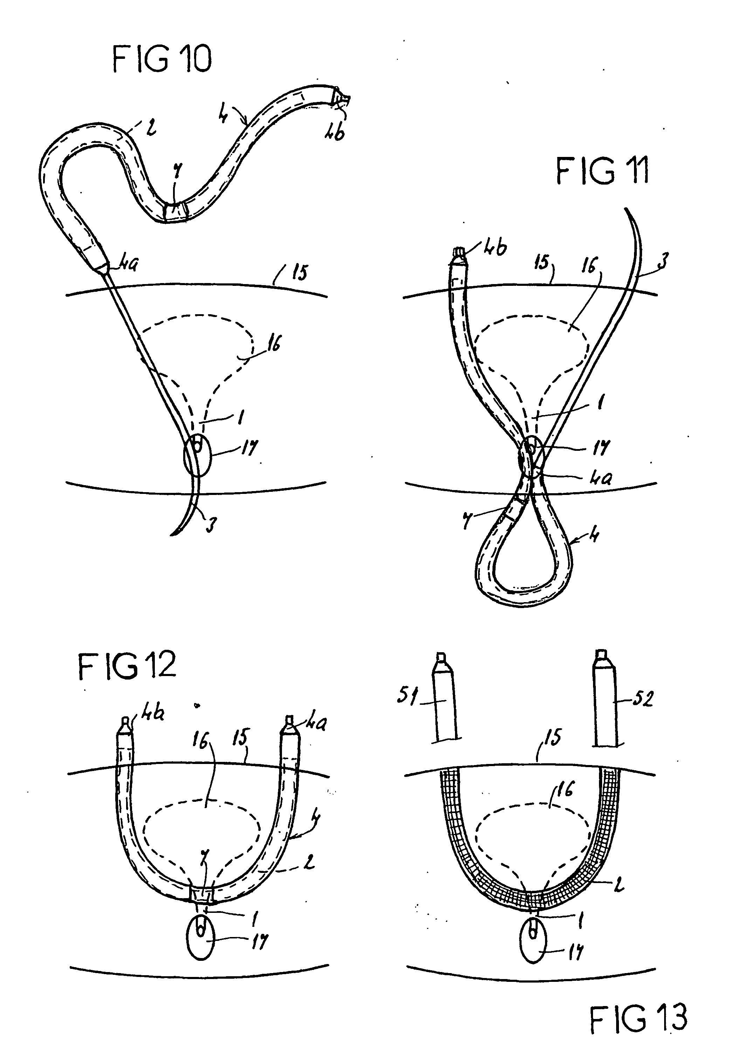

S'agissant de la voie mixte, et en se référant aux figures 10 à 13 :
- on fait pénétrer l'aiguille 3 de haut en bas, du côté droit du patient, en contournant la vessie 16, et en ressortant par le vagin 17 ; cf la figure 10 ;
- on engage ensuite la bande composite 4 dans le trajet droit, par pénétration de l'aiguille 3 de bas en haut, du côté gauche, en contournant la vessie 16 ; cf figure 11 ;
- on engage ainsi la bande composite 4 dans le trajet gauche ; on déconnecte l'aiguille 3 de la bande composite 4 ; on déchire transversalement le moyen de liaison 7, par cisaillement du fourreau extérieur 71, et on extrait de ce dernier à l'extérieur du vagin 17 (cf figure 12) ;
- par traction sur les deux embouts 9, on extrait les deux parties 51 et 52 de la gaine 5, et on libère la bandelette 2 de soutènement dans son trajet.

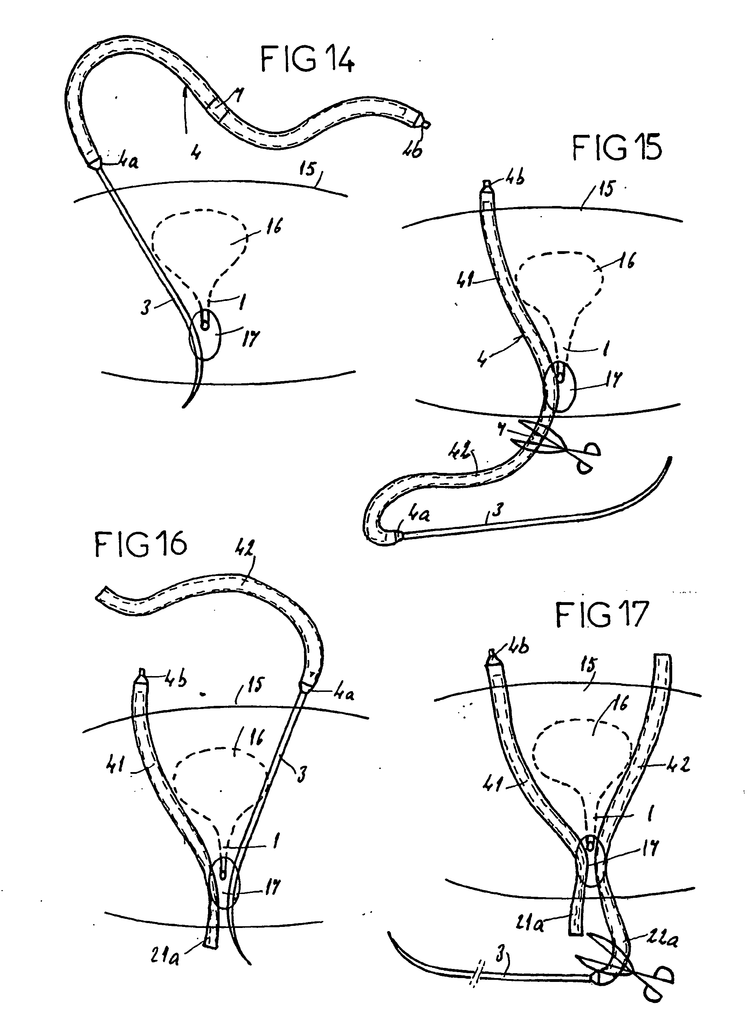

S'agissant de la voie haute, et en se référant aux figures 14 à 21 :
- on fait pénétrer l'aiguille 3 de haut en bas, du côté droit du patient, à partir de la paroi abdominale 15, en contournant la vessie 16, et en ressortant par le vagin 17 (cf figure 14) ;
- on engage ainsi la bande composite 4 dans le trajet droit ; lorsque le moyen de liaison 7 est à l'extérieur du vagin 17, on coupe la bande composite 4 de part et d'autre de ce dernier, de manière à déterminer deux parties, une partie 41 demeurant dans le trajet droit, et une partie 42 à l'extérieur du vagin 17 (cf figure 15) ;
- on fait ensuite pénétrer l'aiguille 3 connectée à la partie 42 de la bande composite 4, de haut en bas à partir de la paroi abdominale 15, du côté gauche, en contournant la vessie 16 (cf figure 16) ;
- on engage ainsi la partie 42 de la bande composite 4 dans le trajet gauche puis on coupe la portion émergente de la partie 42 de la bande composite 4, de manière à séparer l'embout 9 correspondant du reste de la bande 4 (cf figure 17) ;
- en tirant sur les extrémités 21 a et 22a de la bandelette 2 de soutènement, on dénude ces dernières par rapport aux deux parties 52 et 51 respectivement de la gaine 5 ; on peut ainsi présenter la pièce 10 de jonction, et procéder à la préhension des deux extrémités 21a et 22a (cf figure 18) ;
- la pièce 10 de jonction réunit ainsi, de manière sécurisée, les deux extrémités 21a et 22a de la bandelette de soutènement 2, à l'extérieur du vagin 17, comme montré à la figure 19 ;
- puis on procède à une traction suspubienne sur les parties droite 41 et gauche 42 de la bande composite 4, de façon à amener la pièce 10 de jonction en position sous-urétrale : puis on tire sur les deux parties 52 et 51 de la gaine 4, de façon à libérer la bandelette de soutènement 2 ; cf figure 20.
- la bandelette 2 se trouve ainsi en position rétropubienne et para-vésicale, tandis que la pièce 10 de jonction, atraumatique, se trouve en position sous-urétrale (cf figure 21).

Comme décrit précédemment, on constate qu'un ensemble chirurgical selon l'invention peut être utilisé quelle que soit la voie d'abord, et la technique d'intervention choisie par le chirurgien.

Grâce à l'invention, la bandelette 2 de soutènement peut être libérée au dernier moment, après vérification des trajets par cystoscopie.

En cas de trajet erroné, lors du premier passage et/ou lors du second passage, la procédure d'intervention demeure réversible, soit par déconnection de l'aiguille 3 et traction rétrograde de la gaine 5, soit par traction complète de la bande composite 4.

Tant que la gaine 5 n'est pas séparée en ses deux parties 51 et 52, l'ensemble chirurgical selon l'invention est réutilisable autant de fois que nécessaire, et ce jusqu'à ce que le trajet souhaité soit obtenu.

## Revendications

1. Ensemble chirurgical pour soutenir l'urètre (1) chez la femme, comprenant une bandelette (2) de soutènement sous-urétral, une aiguille (3) de traversée percutanée, et une bande composite (4) qui rassemble au moins la bandelette (2) de soutènement sous urétral et une gaine (5) aplatie de protection, la gaine (5) aplatie comprenant, selon sa longueur, deux parties (51, 52) disposées de part et d'autre d'une zone de séparation (6), dans laquelle est disposé un moyen de liaison (7) sécable entre les deux dites parties ; **caractérisé en ce que** :
- ladite bandelette (2) est disposée librement à l'intérieur de cette gaine (5), et **en ce que**
- la bande composite (4) comprend à chacune de ses deux extrémités (4a, 4b), à l'extérieur de la gaine (5), une même partie (81), par exemple une même partie femelle, d'un moyen de connexion (8), verrouillable et déverrouillable, tandis que le talon proximal (31) de l'aiguille (3) de traversée percutanée comprend une autre partie (82), par exemple une partie mâle, dudit moyen de connexion (8).

2. Ensemble selon la revendication 1, **caractérisé en ce que** la bande composite (4) comprend deux embouts (9) comprenant ou incorporant chacun ladite même partie (81) du moyen de connexion (8).

3. Ensemble selon la revendication 1, **caractérisé en ce que** le moyen de connexion (8) comprend deux éléments, l'un mâle (82) et l'autre femelle (81) encliquetables l'un dans l'autre.

4. Ensemble selon la revendication 3, **caractérisé en ce que** le talon proximal (31) de l'aiguille (3) de traversée cutanée est spatulé et chaque embout (9) de la gaine aplatie (5) comprend une fente (83) adaptée pour la pénétration dudit talon proximal spatulé (31), avec un moyen de retenue (84) dudit talon, verrouillable ou déverrouillable manuellement.

5. Ensemble selon la revendication 4, **caractérisé en ce que** le talon proximal (31) de l'aiguille de traversée cutanée (3) comprend une fente traversante (85) pour la pénétration du moyen de retenue (84).

6. Ensemble selon la revendication 1, **caractérisé en ce que** le moyen de liaison (7) sécable, comprend un fourreau extérieur (71) adhésif, par exemple déchirable par cisaillement, rassemblant les deux extrémités intérieures (51a, 52a) des deux parties (51, 52) de la gaine aplatie (5), respectivement adjacentes à la zone de séparation (6).

7. Ensemble selon la revendication 1, **caractérisé en ce qu'**il comprend, en outre, une pièce (10) plate et souple de jonction de deux morceaux (21, 22) de la bandelette (2) de soutènement, obtenus par découpe transversale de cette dernière, ladite pièce comprenant deux moyens opposés (11) de préhension des deux extrémités adjacentes (21a, 22a) des deux morceaux respectivement.

8. Ensemble selon la revendication 7, **caractérisé en ce que** chaque moyen (11) de préhension comprend un moyen de serrage (12) d'une extrémité (21a, 22a) de la bandelette (2) de soutènement.

9. Ensemble selon la revendication 7, **caractérisé en ce que** chaque moyen (11) de préhension comprend un moyen (13) d'agrafage d'une extrémité (21a, 22a) de la bandelette (2) de soutènement, par exemple un pion (13a) pour traverser la bandelette avec une tête (13b) encliquetable sur ledit pion.

10. Ensemble selon la revendication 1, **caractérisé en ce que** la bandelette (2) de soutènement comprend un tissu tricoté ajouré indémaillable, obtenu avec des monofilaments ou multifilaments en matériau synthétique biocompatible, par exemple en polypropylène ou polyester.

11. Ensemble selon la revendication 1, **caractérisé en ce que** bandelette de soutènement (2) est réalisée en tout ou en partie en tissu ou matériau biologique, par exemple en collagène.

12. Ensemble selon la revendication 1, **caractérisé en ce que** la gaine de protection (5) est obtenue à partir d'un matériau synthétique à faible coefficient de frottement, par exemple en PTFE.

## Patentansprüche

1. Chirurgische Unterstützungsanordnung für die weibliche Harnröhre, umfassend einen suburethralen Unterstützungsstreifen (2), eine die Haut durchdringende Nadel (3) und ein Verbundband (4), welches zumindest den suburethralen Unterstützungsstreifen (2) und eine flache Schutzhülle (5) umfasst, wobei die flache Hülle (5) ihrer Länge nach zwei Teilstücke (51, 52) aufweist, welche auf beiden Seiten einer Trennungszone (6) angeordnet sind, in welcher ein Verbindungsmittel (7) angeordnet ist, welches zwischen den beiden Teilstücken teilbar ist, **dadurch gekennzeichnet, dass**
- der Streifen (2) frei beweglich im Inneren der Hülle (5) angebracht ist und dass
- das Verbundband (4) an jedem seiner beiden Enden (4a, 4b), am Äußeren der Hülle (5), ein gleiches Teil (81) aufweist, beispielsweise ein gleiches Buchsenteil eines verriegelbaren oder nicht verriegelbaren Verbindungsmittels, während der proximale Schaft (31) der die Haut durchdringenden Nadel (3) einen anderen Teil (82) aufweist, beispielsweise ein Steckerteil des Verbindungsmittels (8).

2. Anordnung nach Anspruch 1, **dadurch gekennzeichnet, dass** das Verbundband (4) zwei Endstücke (9) aufweist, welche jeweils das gleiche Teil (81) des Verbindungsmittels (8) aufweisen oder dieses enthalten.

3. Anordnung nach Anspruch 1, **dadurch gekennzeichnet, dass** das Verbindungsmittel (8) zwei Elemente aufweist, ein Steckerelement (82) und ein Buchsenelement (81), welche ineinander einrasten können.

4. Anordnung nach Anspruch 3, **dadurch gekennzeichnet, dass** der proximale Schaft (31) der die Haut durchdringenden Nadel (3) spatenförmig ist und dass jedes Endstück (9) der flachen Hülle (5) einen Schlitz (83) aufweist, der zur Einführung des spatenförmigen proximalen Schafts ausgebildet ist, mit manuell verriegelbaren oder entriegelbaren Mitteln zum Zurückhalten (84) des Schafts.

5. Anordnung nach Anspruch 4, **dadurch gekennzeichnet, dass** der proximale Schaft (31) der die Haut durchdringenden Nadel (3) eine durchgehende Nut (85) für die Einführung der Rückhaltemittel (84) aufweist.

6. Anordnung nach Anspruch 1, **dadurch gekennzeichnet, dass** das teilbare Verbindungsmittel (7) eine äußere Klebehülse (71) aufweist, welche beispielsweise durch Scherwirkung zerrissen werden kann und welche die zwei inneren Enden (51a, 52a) der beiden Teile (52, 52) der flachen Hülle (5) verbindet, welche jeweils an die Trennungszone (6) angrenzen.

7. Anordnung nach Anspruch 1, **dadurch gekennzeichnet, dass** sie ferner ein Teil (10) aufweist, welches flach und gelenkig zur Verbindung zweier Fragmente (21, 22) des Unterstützungsstreifens (2) ausgebildet ist, welche durch Durchschneiden des Unterstützungsstreifens erhalten werden können, wobei das Teil zwei gegenüberliegende Mittel (11) jeweils zum Greifen der zwei angrenzenden Enden (21a, 22a) der zwei Fragmente aufweist.

8. Anordnung nach Anspruch 7, **dadurch gekennzeichnet, dass** jedes Greifmittel (11) ein Mittel zum Einklemmen (12) des einen Endes (21a, 22a) des Unterstützungsstreifens (2) aufweist.

9. Anordnung nach Anspruch 7, **dadurch gekennzeichnet, dass** jedes Greifmittel (11) ein Mittel (13) zum Verklemmen des einen Endes (21a, 22a) des Unterstützungsstreifens (2) aufweist, beispielsweise einen Stift (13a) zum Durchdringen des Streifens, mit einem Kopf (13b), welcher über den Stift eingerastet werden kann.

10. Anordnung nach Anspruch 1, **dadurch gekennzeichnet, dass** der Unterstützungsstreifen (2) ein maschenfestes gewirktes Ajourgewebe aufweist, welches mit Mono- oder Multifilamenten aus einem biokompatiblen synthetischem Material gewonnen werden kann, beispielsweise aus Polypropylen oder Polyester.

11. Anordnung nach Anspruch 1 **dadurch gekennzeichnet, dass** der Unterstützungsstreifen (2) vollständig oder teilweise aus einem biologischen Material oder Gewebe hergestellt ist, beispielsweise aus Collagen.

12. Anordnung nach Anspruch 1 **dadurch gekennzeichnet, dass** die Schutzhülle (5) aus einem synthetischen Material mit einem geringen Reibungskoeffizienten gewonnen wird, beispielsweise aus PTFE.

## Claims

1. Surgical assembly for supporting the female urethra (1), comprising a suburethral support strip (2), a percutaneous needle (3), and comprises a composite band (4) which includes at least the suburethral support strip (2) and a flat protective sheath (5), the flat sheath (5) comprising, along its length, two parts (51, 52) arranged on either side of a zone (6) of separation in which there is a breakable linking means (7) arranged between the two said parts; **characterized in that**
- said strip (2) is arranged freely inside this sheath (5), and **in that**
- at each of its two ends (4a, 4b), outside the sheath (5), the composite band (4) comprises one part (81), for example a female part, of a lockable and unlockable connecting means (8), while the proximal heel (31) of the percutaneous needle (3) comprises another part (82), for example a male part, of said connecting means (8).

2. Assembly according to Claim 1, **characterized in that** the composite band (4) comprises two endpieces (9) which each comprise or incorporate said one part (81) of the connecting means (8).

3. Assembly according to Claim 1, **characterized in that** the connecting means (8) comprises two elements, one male (82) and the other female (81), which can be snapped into one another.

4. Assembly according to Claim 3, **characterized in that** the proximal heel (31) of the percutaneous needle (3) is spatulate and each end-piece (9) of the flat sheath (5) comprises a slot (83) designed for the penetration of said spatulate proximal heel (31), with a manually lockable or unlockable means (84) for retention of said heel.

5. Assembly according to Claim 4, **characterized in that** the proximal heel (31) of the percutaneous needle (3) comprises a transverse slot (85) for the penetration of the retention means (84).

6. Assembly according to Claim 1, **characterized in that** the breakable linking means (7) comprises an outer adhesive sleeve (71) which can be torn off for example by shearing and includes the two inner ends (51a, 52a) of the two parts (51, 52) of the flat sheath (5) respectively adjacent to the zone (6) of separation.

7. Assembly according to Claim 1, **characterized in that** it additionally comprises a flat and flexible component (10) for joining two pieces (21, 22) of the support strip (2) which have been obtained by cutting this strip transversely, said component comprising two opposite means (11) for respectively gripping the two adjacent ends (21a, 22a) of the two pieces.

8. Assembly according to Claim 7, **characterized in that** each gripping means (11) comprises a means (12) for clamping one end (21a, 22a) of the support strip (2).

9. Assembly according to Claim 7, **characterized in that** each gripping means (11) comprises a means (13) for fastening one end (21a, 22a) of the support strip (2), for example a pin (13a) which passes through Lhe strip and a head (13b) which can be snapped onto said pin.

10. Assembly according to Claim 1, **characterized in that** the support strip (2) comprises an openwork knit which is unable to unravel and is obtained from monofilaments or multifilaments of a biocompatible synthetic material, for example polypropylene or polyester.

11. Assembly according to Claim 1, **characterized in that** the support strip (2) is made wholly or partly of biological Lissue or material, for example collagen.

12. Assembly according to Claim 1, **characterized in that** the protective sheath (5) is obtained from a synthetic material with a low coefficient of friction, for example PTFE.
